# EUROPEAN PATENT APPLICATION

(11) **EP 3 736 345 A1**
(43) Date of publication of application: **11.11.2020**
(21) Application number: 19382368.9
(22) Date of filing: 10.05.2019
(51) Int. Cl.: C12Q 1/6886

(54) **GENOMIC PREDICTORS OF AGGRESSIVE MICROPAPILLARY BLADDER CANCER**

(71) Applicant: Fundació Institut Mar d'Investigacions Mèdiques (IMIM), 08003 Barcelona (ES); Bowden, Michaela, Arlington 02476 (US)
(72) Inventor: BOWDEN, Michaela, ARLINGTON, MA 02476 (US); BELLMUNT MOLINS, Joaquim, 08003 BARCELONA (ES)
(74) Representative: ZBM Patents - Zea, Barlocci & Markvardsen

(57) **Abstract**

The present invention provides *in vitro* method for the diagnosis and/or the prediction of the progression of high grade T1 (HGT1) micropapillary bladder cancer (MPBC), the method comprising quantifying the expression product of each one of FABP3, CD36 and RAET1E genes in an isolated sample from the subject.

This combination of markers provides a valuable tool which allows significantly discriminating a HGT1 NMIBC patient which could benefit from and early RC from one which could not need it. Consequently, this set of markers can have potential applications in the clinical management of MPBC cancer patients, in terms of recommending an early radical cystectomy in these patients at high risk of progression.

## Description

### Technical Field

The present invention relates to the field of bladder cancer. In particular, the present invention provides a new method for predicting the aggressiveness of a micropapillary variant in non-muscle invasive bladder cancer (NMIBC) patients.

### Background Art

Non-muscle invasive bladder cancer (NMIBC) is a heterogeneous disease comprising almost 75% of all bladder cancer patients. Amongst several of the potential adverse features, the presence of a variant histology (ie. micropapillary, nested, plasmacytoid, sarcomatoid) is associated with poor outcome.

Micropapillary bladder cancer (MPBC) variant was first described as a distinct histological subtype of bladder carcinoma (BC) in 1994. This rare variant represents approximately 0.01-2.2% of urothelial tumors and has been associated with a higher stage at diagnosis and increased risk of metastatic disease, even if it comprises only a fraction of the overall tumor volume.

High-grade T1 (HGT1) with micropapillary variant is a subgroup of NMIBC considered to be highly aggressive. It is unclear whether micropapillary HGT1 can be treated as conventional NMIBC - with bladder-preserving therapies using TURBT and BCG -, or if these patients should undergo early radical cystectomy (RC). In the largest retrospective report of MPBC, including 31 MP-HGT1, Kamat *et al.* at MD Anderson found that overall prognosis was poor, and made the case for early radical cystectomy in any NMIBC with a micropapillary component (Kamat A. M. et al., 2007). Conversely, Spaliviero *et al.* challenged this recommendation as they observed no significant differences in 5-year disease-specific mortality or incidence of metastasis in their MSKCC series of 36 non-muscle invasive MPBC (Spaliviero M et al., 2014). This lack of agreement might be explained by differences on the biology and molecular characteristics of MPBC (Kamat A.M. et al., 2007).

There are currently only two global RNA expression studies of MPBC in MIBC (Warrick J. I. et al., 2019; and Sui W. et al., 2016), and neither provides clinical recommendations based on genomic profiling.

Radical cystectomy (RC) is the standard treatment for patients with muscle-invasive bladder cancer, and it is also a valid option for selected patients with high-grade non-muscle-invasive bladder cancer, either as a primary treatment modality or for recurrent or refractory tumours after bladder-conserving regimens. Radical cystectomy entails simultaneous surgery on the urinary tract, intestines (for urinary diversion), and lymph nodes; hence, complications frequently occur after this extensive procedure.

An appropriate management with RCs in HGT1 patients is sometimes required to alleviate and improve patient's quality life and survival.In spite of performing such risky intervention, however, RC is not always efficient in HGT1 patients. Thus, HGT1 patient's life can be seriously compromised with an intervention that, for the particular disease profile, is sometimes unprofitable.

Consequently, in spite of the efforts made, there is a need to identify patients at high risk for progression in HGT1 having specific variants like MPBC which can benefit from an early RC.

### Summary of Invention

The inventors have found that the analysis of the expression of certain genes can provide useful information about the aggressiveness and time to progression (TTP) of HGT1 MPBC.

The time to progression can be understood as the length of time from the date of diagnosis or the start of standart treatment for the disease (in the present case TURBT plus BCG) until the disease starts to get worse (progression to MIBC), requiring a RC or spread to other parts of the body (metastasis) or death. Measuring the time to progression is one way to see how well a new treatment can work. The shorter is the TTP value, the more advisable first therapeutic approach is RC-based.

The present inventors herein provide a set of genes which is significantly associated with TTP (see Table 3) and which allows defining a more aggressive phenotype of HGT1 MPBC - NMIBC. In particular, CD36, FAPB3 and RAETE1 were determined in patients which were already diagnosed with the disease at the time of the TURBT, and it was found that all three were associated with short TTP: high expression of each one of FABP3 and CD36 were associated with shorter TTP p=0.13 and p=0.02, respectively, and low expression of RAET1E with shorter TTP (P=0.0005). Surprisingly, when all three genes were taken into consideration when performing the statistical analysis, it was found that the p value was substantially reduced to 0.0001 and was significantly associated to a shorter TTP (see below); which means that the combination synergistically significantly differentiates this population of patients from others.

In view of the above, the clinician, faced with the need of determining whether the patient suffering HGT1 MPBC - NMIBC, can benefit from a RC, has a precise 3-marker based tool: if the clinician found that FABP3 and CD36 are highly expressed and RAETE1 low expressed, this would be indicative that the RC is a good therapeutic approach to the patient.

Therefore, this combination of markers provides a valuable tool which allows significantly discriminating a HGT1 MPBC - NMIBC patient which could benefit and positively respond to early RC from one which could not. Consequently, this set of markers can have potential applications in the clinical management of MPBC cancer patients, in terms of radical cystectomy recommendation.

Thus, in first aspect the present invention provides an *in vitro* method for the diagnosis and/or the prediction of the progression of high grade T1 (HGT1) micropapillary bladder cancer (MPBC), the method comprising quantifying the expression product of each one of FABP3, CD36 and RAET1E genes in an isolated sample from the subject.

The present inventors have also found that these markers provide diagnostic information, as they were at least 2-fold higher with respect to the corresponding reference values (Table 2 below).

The analysis of these 3 differentially expressed genes (FABP3, CD36 and RAET1E) allowed to create a risk index that could help to identify patients with MPBC - HGT1 with a higher risk of progression (i.e., with a shorter TTP), wherein RC could be recommended as a first treatment option.

In a second aspect the present invention provides an *in vitro* method for deciding or recommending a radical cystectomy-based medical regimen to a patient with HGT1 MPBC, the method comprising the steps of (i) quantifying the gene expression product of FABP3, CD36 and RAET1E in an isolated biological sample of the patient, (ii) comparing the amount of each expression product with the reference value for each one of the markers, and (iii) recommending a radical cystectomy if the gene expression products of FABP3 and CD36 are higher than the corresponding reference values, and the gene expression product of RAET1E is lower than the corresponding reference value.

In a third aspect the invention provides an *in vitro* method for deciding or recommending a medical regimen to a patient with HGT1 MPBC, the method comprising the steps of (i) quantifying the gene expression product of FABP3, CD36 and RAET1E in an isolated biological sample of the patient, (ii) comparing the amount of each expression product with the reference value for each one of the markers, and (iii) recommending the standard therapy of transurethral resection of bladder tumor (TURBT)and intravesical bacillus Calmette-Guerin (BCG) therapy if the gene expression product of FABP3 or CD36 is equal or lower than the corresponding reference value, and/or the gene expression product of RAET1E is equal or higher than the corresponding reference value.

In a fourth aspect the present invention provides the combined use of FABP3, CD36 and RAET1E as a marker of diagnosis or prediction of progression of HGT1 MPBC.

In a fifth aspect the present invention provides the combined use of FABP3, CD36 and RAET1E for deciding or recommending a medical regimen to a patient with HGT1 MPBC.

And, finally, in a sixth aspect the present invention provides the use of means for quantifying the gene expression product of each one of FABP3, CD36 and RAET1E in any of the methods as defined in the first, second and third aspects of the invention.

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

The present invention provides new biomarkers for the diagnosis or prediction of progression of high grade T1 (HGT1) micropapillary bladder cancer (MPBC).

The term "diagnosis" is known to the person skilled in the art. As used herein "diagnosis" is understood as becoming aware of a particular medical condition complication or risk in a subject; the determination of the nature of the disease or condition; or the distinguishing of one disease or condition from another. It refers both to the process of attempting to determine or identify the possible disease or disorder, and to the opinion reached by this process. A diagnosis, in the sense of diagnostic procedure, can be regarded as an attempt at classification of an individual's condition into separate and distinct categories that allow medical decisions about treatment and prognosis to be made. Subsequently, a diagnostic opinion is often described in terms of a disease or other condition. However, a diagnosis can take many forms. It might be a matter of detecting the presence and naming the disease, lesion, dysfunction or disability. It might be an exercise to attribute a category for management or for prognosis. It may indicate either degree of abnormality on a continuum or kind of abnormality in a classification.

As it has been explained above, the methods provided by the present invention are based on quantifying the amount of expression product of the genes FABP3, CD36 and RAETE1.

In one embodiment of any of the methods provided by the present invention, optionally in combination with any of the embodiments provided above or below, the expression product is mRNA. In an alternative embodiment of any of the methods provided by the invention, optionally in combination with any of the embodiments provided above or below, the expression product is protein.

In the present invention by "quantifying" the expression product has to be understood determining the amount of the product in an isolated test sample. There are well-known routine techniques for quantifying a particular product, the technique being dependent on the nature of the expression product type.

In one embodiment of any of the methods provided by the present the invention, optionally in combination with any of the embodiments provided above or below, it is determined the amount of mRNA for each one of the gene markers provided by the invention via polymerase chain reaction (using, for example, oligonucleotide primers). In particular embodiments of the invention, optionally in combination with any of the embodiments provided above or below, RT-PCR can be used to amplify the mRNA for protein cancer markers for detection and analysis. Other embodiments of the invention, optionally in combination with any of the embodiments provided above or below, use quantitative RT-PCR to quantitatively determine amount of mRNA for protein cancer markers. Further embodiments of the invention use real time RT-PCR for quantification and analysis.

Alternatively, in another embodiment of any of the methods provided by the present invention, optionally in combination with any of the embodiments provided above or below, it is determined the amount of mRNA for each one of the gene markers provided by the invention via a hybridization technique, employing oligonucleotide probes.

When using mRNA quantification, the method may be carried out by combining isolated mRNA with reagents to convert to cDNA according to standard methods well known in the art, treating the converted cDNA with amplification reaction reagents (such as cDNA PCR reaction reagents) in a container along with an appropriate mixture of nucleic acid primers; reacting the contents of the container to produce amplification products; and analyzing the amplification products to detect the presence of one or more of the polynucleotide cancer markers in the sample. The analysis step may be further accomplished by quantitatively detecting the presence of polynucleotide cancer markers in the amplification product, and comparing the quantity of marker detected against a panel of expected values for the known presence or absence of such markers in normal and malignant tissue derived using similar primers.

Alternatively, the RNA quantification can be performed by fluorescence-based technique, using fluorescein excitation and emission wavelengths of appropriate reagents which are commercially available (such as Quant-iT™ RiboGreen® RNA reagent). To this end, commercially available fluorescent RNA-binding dies and a device such as fluorescence microplate reader, standard spectrofluorometer, or filter fluorometer can be used.

In any of the embodiments provided above or below, for any of the aspects of the invention, the level of expression is determined at the protein level. In this embodiment, the protein marker(s) include, but do not limit to, native-sequence polypeptides, isoforms, chimeric polypeptides, all homologs, fragments, and precursors of the markers, including modified forms of the polypeptides and derivatives thereof.

In any of the embodiments provided above or below, the level of expression is determined by immunochemistry.

The term "immunochemistry" as used herein refers to a variety of techniques for detecting antigens (usually proteins and peptides, and in the present case any of the proteins listed above alone or in combination) in a sample by exploiting the principle of antibodies binding specifically to said antigens. Visualising an antibody-antigen interaction can be accomplished in a number of ways. In the most common instance, an antibody is conjugated to an enzyme, such as peroxidase, that can catalyse a colour-producing reaction. Alternatively, the antibody can also be tagged to a fluorophore, such as fluorescein or rhodamine. The immunochemistry technique can be direct or indirect. The direct method is a one-step staining method and involves a labeled antibody (e.g. FITC-conjugated antiserum) reacting directly with the antigen. While this technique utilizes only one antibody and therefore is simple and rapid, the sensitivity is lower due to little signal amplification, such as with indirect methods, and is less commonly used than indirect methods. The indirect method involves an unlabeled primary antibody (first layer) that binds to the target antigen in the sample and a labeled secondary antibody (second layer) that reacts with the primary antibody. This method is more sensitive than direct detection strategies because of signal amplification due to the binding of several secondary antibodies to each primary antibody if the secondary antibody is conjugated to the fluorescent or enzyme reporter.

Further amplification can be achieved if the secondary antibody is conjugated to several biotin molecules, which can recruit complexes of avidin-, streptavidin or Neutravidin-enzyme. The indirect method, aside from its greater sensitivity, also has the advantage that only a relatively small number of standard conjugated (labeled) secondary antibodies needs to be generated. With the direct method, it would be necessary to label each primary antibody for every antigen of interest. It must be borne in mind that immunochemistry techniques can also be used to detect certain nucleic acid sequences if a tagged nucleic acid probe (designed to specifically bind to a certain target nucleic acid sequence) can later on be detected with a labelled antibody. Thus, the detection of the protein could be performed by using a tagged nucleic acid designed to bind a specific sequence of the target protein RNA, and then detecting said tagged nucleic acid with a labelled antibody which selectively binds to the tag.

Immunoassay procedures suitable include enzyme-linked immunosorbent assays (ELISA), enzyme immunodot assay, agglutination assay, antibody-antigen-antibody sandwich assay, antigen-antibody-antigen sandwich assay, immunocromatography, or other immunoassay formats well-known to the ordinarily skilled artisan.

In one embodiment, in combination with any of the embodiments provided above or below, the level of expression of protein is determined by an immunoassay.

In another embodiment, in combination with any of the embodiments provided above or below, the level of expression of protein is determined by ELISA.

Alternatively, the level of expression of protein can be determined by bioluminescence, fluorescence, chemiluminescence, electrochemistry, or mass spectrometry.

In another embodiment, in combination with any of the embodiments provided above or below, the level of expression of protein is determined using an antibody or a fragment thereof able to bind to the target protein(s).

The term "antibody or a fragment thereof able to bind to the target protein(s)" is to be understood as any immunoglobulin or fragment thereof able to selectively bind the target protein. It includes monoclonal and polyclonal antibodies. The term "fragment thereof encompasses any part of an antibody having the size and conformation suitable to bind an epitope of the target protein. Suitable fragments include F(ab), F(ab') and Fv. An "epitope" is the part of the antigen being recognized by the immune system (B-cells, T-cells or antibodies).

The antibodies used for specific detection can be polyclonal or monoclonal. There are well known means in the state of the art for preparing and characterizing antibodies. Methods for generating polyclonal antibodies are well known in the prior art. Briefly, one prepares polyclonal antibodies by immunizing an animal with the protein; then, serum from the immunized animal is collected and the antibodies isolated. A wide range of animal species can be used for the production of the antiserum. Typically the animal used for production of antisera can be a rabbit, mouse, rat, hamster, guinea pig or goat.

Moreover, monoclonal antibodies (MAbs) can be prepared using well-known techniques. Typically, the procedure involves immunizing a suitable animal with the protein associated with the disease. The immunizing composition can be administered in an amount effective to stimulate antibody producing cells. Methods for preparing monoclonal antibodies are initiated generally following the same lines as the polyclonal antibody preparation. The immunogen is injected into animals as antigen. The antigen may be mixed with adjuvants such as complete or incomplete Freund's adjuvant. At intervals of two weeks, approximately, the immunization is repeated with the same antigen.

In another embodiment of any of the methods provided by the present invention, optionally in combination with any of the embodiments provided above or below, it is further quantified at least a gene product selected from the following list: COL4A4, LCN2, KL, ABCA13, CYP1B1, ANPEP, DACH2, KRTAP5-9, CAPNS2, KRTAP5-8, NPAP1, GDPD2, HECTD2-AS1, SNHG8, GABBR2, C12orf75, PKP1, CEACAM6, CRTAC1, IGF2, MMP7, CHST3, TNFRSF11B, and combinations thereof.

The following table provides summarized information of the markers referred in the methods provided by the invention:

**Table 1**

| **Gene** | **Description** | **Genebank number** |
|---|---|---|
| COL4A4 | collagen, type IV, alpha 4 | Gene ID: 1286, updated on 19-Mar-2019 |
| LCN2 | lipocalin 2 | Gene ID: 3934, updated on 6-Apr-2019 |
| KL | Klotho | Gene ID: 9365, updated on 7-Apr-2019 |
| ABCA13 | ATP-binding cassette, sub-family A (ABC1), member 13 | Gene ID: 154664, updated on 13-Feb-2019 |
| FABP3 | fatty acid binding protein 3 | Gene ID: 2170, updated on 13-Feb-2019 |
| CYP1B1 | cytochrome P450 family 1 subfamily B member 1 | Gene ID: 1545, updated on 28-Mar-2019 |
| ANPEP | alanyl aminopeptidase | Gene ID: 290, updated on 13-Feb-2019 |
| DACH2 | dachshund family transcription factor 2 | Gene ID: 117154, updated on 13-Feb-2019 |
| KRTAP5-9 | keratin-associated protein 5-9 | Gene ID: 3846, updated on 13-Feb-2019 |
| CAPNS2 | calpain small subunit 2 | Gene ID: 84290, updated on 13-Feb-2019 |
| KRTAP5-8 | keratin-associated protein 5-8 | Gene ID: 57830, updated on 13-Feb-2019 |
| NPAP1 | nuclear pore associated protein 1 | Gene ID: 23742, updated on 13-Feb-2019 |
| GDPD2 | glycerophosphodiester phosphodiesterase domain containing 2 | Gene ID: 54857, updated on 13-Feb-2019 |
| HECTD2-AS1 | HECTD2 antisense RNA 1 | Gene ID: 100188947, updated on 13-Feb-2019 |
| SNHG8 | small nucleolar RNA host gene 8 | Gene ID: 100093630, updated on 13-Feb-2019 |
| GABBR2 | gamma-aminobutyric acid (GABA) B receptor, 2 | Gene ID: 9568, updated on 13-Feb-2019 |
| C12orf75 | chromosome 12 open reading frame 75 | Gene ID: 387882, updated on 7-Apr-2019 |
| PKP1 | plakophilin 1 | Gene ID: 5317, updated on 13-Feb-2019 |
| CEACAM6 | carcinoembryonic antigen-related cell adhesion molecule 6 | Gene ID: 4680, updated on 13-Feb-2019 |
| CRTAC1 | cartilage acidic protein 1 | Gene ID: 55118, updated on 13-Feb-2019 |
| CD36 | platelet glycoprotein IV | Gene ID: 948, updated on 9-Apr-2019 |
| IGF2 | insulin-like growth factor 2 | Gene ID: 3481, updated on 9-Apr-2019 |
| MMP7 | matrix metallopeptidase 7 | Gene ID: 4316, updated on 2-Apr-2019 |
| RAET1E | retinoic acid early transcript 1E | Gene ID: 135250, updated on 13-Feb-2019 |
| CHST3 | carbohydrate sulfotransferase 3 | Gene ID: 9469, updated on 13-Feb-2019 |
| TNFRSF11 B | TNF receptor superfamily member 11b | Gene ID: 4982, updated on 2-Apr-2019 |

In another embodiment of any of the methods provided by the present invention, optionally in combination with any of the embodiments provided above or below, it is further quantified the gene product from COL4A4, LCN2, KL, ABCA13, CYP1B1, ANPEP, DACH2, KRTAP5-9, CAPNS2, KRTAP5-8, NPAP1, GDPD2, HECTD2-AS1, SNHG8, GABBR2, C12orf75, PKP1, CEACAM6, CRTAC1, IGF2, MMP7, CHST3 and TNFRSF11B genes.

In another embodiment of any of the methods provided by the present invention, optionally in combination with any of the embodiments provided above or below, the method further comprises the step of comparing the expression of each one of the gene product(s) with a reference value.

In the present invention, the term "reference value" referred to in the methods of the invention is to be understood as a predefined value of a given molecular marker, in the present case any of the markers listed in the first or second aspects as well as in particular embodiments, which is derived from the levels of said molecular marker in a sample or group of samples. If the level of expression is determined at the protein level, then the "reference value" is a predefined value of protein quantity, whereas if the level of expression is determined at the mRNA level, then the "reference value" is a predefined value of mRNA quantity. The samples are taken from a subject or group of subjects wherein the presence, absence, stage, or course of the disease has been properly performed previously. This value is used as a threshold to discriminate subjects wherein the condition to be analyzed is present from those wherein such condition is absent (i.e. subject having MPBC - HGT1 cancer from subjects with HGT1 free of MPBC component), to determine the stage of the disease and the aggressiveness of the cancer. This reference value is also useful for determining whether the subject will positively respond to a conventional treatment or requires early RC. The subject or subjects from whom the "reference value" is derived may include subject/s wherein the condition is absent, subject/s wherein the condition is present, or both. The skilled person in the art, making use of the general knowledge, is able to choose the subject or group of subjects more adequate for obtaining the reference control level for each of the methods of the present invention. Methods for obtaining the reference value from the group of subjects selected are well-known in the state of the art (Burtis C. A. et al., 2008, Chapter 14, section "Statistical Treatment of Reference Values") In a particular case "reference control level" is a cut-off value defined by means of a conventional ROC analysis (Receiver Operating Characteristic analysis). As the skill person will appreciate, optimal cut-off value will be defined according to the particular applications of the diagnostic or prognostic method: purpose, target population for the diagnosis or prognosis, balance between specificity and sensibility, etc. The reference value should be determined for each one of the expression gene products referred in the methods of the invention.

In one embodiment of the first aspect of the invention, optionally in combination with any of the embodiments provided above or below, the method is a method of diagnosing HGT1 with MPBC component and the reference value for each one of the gene is defined as the value in a conventional HGT1 sample. In the present invention, by "conventional HGT1" is understood a sample having less than 25% of micropapillar variant. The absence, presence and extension of the micropapillar variant is determined by the pathologist, when he visually analyses the sample by microscopy. In one embodiment of the first aspect of the invention, optionally in combination with any of the embodiments provided above or below, the conventional HGT1 sample is one with no micropapillar variant.

In another embodiment of the first aspect of the invention, optionally in combination with any of the embodiments provided above or below, the method comprises the steps of: (i) comparing the amount of each of the expression product genes with the corresponding reference value, and (ii) diagnosing subject as having HGT1 MPBC if the expression gene product for each one of the markers is at least 2-fold higher with respect to the corresponding reference value for each marker. In another embodiment of the first aspect of the invention, optionally in combination with any of the embodiments provided above or below, the method comprises the steps of: (i) comparing the amount of each of the expression product genes CD36, FABP3 and RAETE1, with the corresponding reference value, (ii) quantifying the expression gene product of at least one gene selected from: COL4A4, LCN2, KL, ABCA13, CYP1B1, ANPEP, DACH2, KRTAP5-9, CAPNS2, KRTAP5-8, NPAP1, GDPD2, HECTD2-AS1, SNHG8, GABBR2, C12orf75, PKP1, CEACAM6, CRTAC1, IGF2, MMP7, CHST3 and TNFRSF11B and comparing the amount with the reference value(s) of the corresponding marker(s), and (iii) diagnosing subject as having HGT1 MPBC if the expression gene product for each one of the markers is at least 2-fold higher with respect to the corresponding reference value. In another embodiment of the first aspect of the invention, optionally in combination with any of the embodiments provided above or below, the method comprises the steps of: (i) comparing the amount of each of the expression product genes CD36, FABP3, RAETE1, COL4A4, LCN2, KL, ABCA13, CYP1B1, ANPEP, DACH2, KRTAP5-9, CAPNS2, KRTAP5-8, NPAP1, GDPD2, HECTD2-AS1, SNHG8, GABBR2, C12orf75, PKP1, CEACAM6, CRTAC1, IGF2, MMP7, CHST3 and TNFRSF11B, with the corresponding reference values, and (ii) diagnosing subject as having HGT1 MPBC if the expression gene product for each one of the markers is at least 2-fold higher with respect to the corresponding reference value.

In another embodiment of the first aspect of the invention, optionally in combination with any of the embodiments provided above or below, the method is a method of predicting the progression of HGT1 with MPBC and the reference value is defined as explained above.

In another embodiment of the first aspect of the invention, optionally in combination with any of the embodiments provided above or below, the method comprises the steps of (i) comparing the amount of each one of the expression product genes with the corresponding reference value, and (ii) identifying the subject as having bad prognosis (a short TTP), when the gene expression products of FABP3 and CD36 are higher and the gene expression product of RAET1E is lower, when compared to the respective reference values.

In another embodiment of the methods of the second and third aspects of the invention, optionally in combination with any of the embodiments provided above or below, the method further comprises quantifying the expression gene product of at least one gene selected from: COL4A4, LCN2, KL, ABCA13, CYP1B1, ANPEP, DACH2, KRTAP5-9, CAPNS2, KRTAP5-8, NPAP1, GDPD2, HECTD2-AS1, SNHG8, GABBR2, C12orf75, PKP1, CEACAM6, CRTAC1, IGF2, MMP7, CHST3 and TNFRSF11B. In another embodiment of the methods of the second and third aspects of the invention, optionally in combination with any of the embodiments provided above or below, the method further comprises quantifying the expression gene product of the following genes: COL4A4, LCN2, KL, ABCA13, CYP1B1, ANPEP, DACH2, KRTAP5-9, CAPNS2, KRTAP5-8, NPAP1, GDPD2, HECTD2-AS1, SNHG8, GABBR2, C12orf75, PKP1, CEACAM6, CRTAC1, IGF2, MMP7, CHST3 and TNFRSF11B.

In another embodiment of the methods provided by the present invention, optionally in combination with any of the embodiments provided above or below, the isolated test sample is an isolated bladder tissue sample, urine, blood, serum and plasma. In another embodiment of the methods provided by the present invention, optionally in combination with any of the embodiments provided above or below, the isolated test sample is an isolated bladder tissue sample.

The *in vitro* methods of the invention provide diagnostic and prognostic information as well the recommendation of a particular medical regimen. In one embodiment, the methods of the invention further comprise the steps of (i) collecting the information resulting from performing the methods of the first, second and third aspects of the invention, and (ii) saving the information in a data carrier.

In the sense of the invention a "data carrier" is to be understood as any means that contain meaningful information data for the diagnosis or prognosis of HGT1 carcinoma, such as paper. The carrier may also be any entity or device capable of carrying the prognosis data. For example, the carrier may comprise a storage medium, such as a ROM, for example a CD ROM or a semiconductor ROM, or a magnetic recording medium, for example a floppy disc or hard disk. Further, the carrier may be a transmissible carrier such as an electrical or optical signal, which may be conveyed via electrical or optical cable or by radio or other means. When the prognosis data are embodied in a signal that may be conveyed directly by a cable or other device or means, the carrier may be constituted by such cable or other device or means. Other carriers relate to USB devices and computer archives. Examples of suitable data carrier are paper, CDs, USB, computer archives in PCs, or sound registration with the same information.

In a fourth aspect the present invention provides the combined use of FABP3, CD36 and RAETE1 in the diagnosis or prediction of progression of HGT1 MPBC.

In a fifth aspect the present invention provides the combined use of FABP3, CD36 and RAETE1 in recommending a medical regimen to a patient with HTG1 MPBC. In one embodiment of the fifth aspect of the invention the medical regimen is RC. In an alternative embodiment of the fifth aspect of the invention, the medical regimen is selected using standard TURBT and BCG.

In an embodiment of the fourth and fifth aspects, the isolated test sample is an isolated bladder tissue sample, preferably paraffin-embedded.

In another embodiment of the fourth and fifth aspects, the combined use further includes the quantifying of at least one expression gene product selected from: COL4A4, LCN2, KL, ABCA13, CYP1B1, ANPEP, DACH2, KRTAP5-9, CAPNS2, KRTAP5-8, NPAP1, GDPD2, HECTD2-AS1, SNHG8, GABBR2, C12orf75, PKP1, CEACAM6, CRTAC1, IGF2, MMP7, CHST3 and TNFRSF11B. In another embodiment of the fourth and fifth aspects, the combined use further includes the quantifying of the expression gene products selected from: COL4A4, LCN2, KL, ABCA13, CYP1B1, ANPEP, DACH2, KRTAP5-9, CAPNS2, KRTAP5-8, NPAP1, GDPD2, HECTD2-AS1, SNHG8, GABBR2, C12orf75, PKP1, CEACAM6, CRTAC1, IGF2, MMP7, CHST3 and TNFRSF11B.

In a sixth aspect the present invention provides the use of means for quantifying the expression gene product of each one of FABP3, CD36 and RAETE1, in any of the methods of the invention.

The nature of the means depends on the technique selected to quantify the expression gene product. Details about their nature have been provided above (primers, probes, antibodies, and fluorescent dyes, among others). In one embodiment of the sixth aspect of the invention, the means is a fluorescent RNA-binding dye. In another embodiment of the sixth aspect of the invention, the means forms part of a kit.

The kit may additionally comprise further means (additives, solvents) to visualize the interactions (dipsticks, chemiluminescent reagents, turbidimetric reagents, etc.). Suitable additives, solvents and reagents to visualize the antigen-antibody interaction are disclosed in the examples.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### Examples

### 1. Material and methods

### 1.1. Clinical Cohort

HGT1 urothelial carcinoma of the bladder who underwent TURBT in two different centers (Hospital del Mar and Hospital Vall d'Hebron -HVH- , Barcelona, Spain) between April 2004 and April 2011 were included. Archival formalin-fixed paraffin-embedded (FFPE) tissue specimens were collected at the time of TURBT or RC confirming the diagnosis of MP-NMIBC that was defined as at least 25% of MP component. Study approval was granted by the DFCI, H del Mar and the HVH Ethics Committee. Patients were managed in a uniform manner using TURB and BCG and median follow-up was 7.4 years. Patients with concomitant CIS were excluded.

Clinical outcome was classified as: non-recurrent disease (good outcome, GO), recurrent disease (R) or progression to MI (muscle invasive) or metastasis (PD) <4 years. Time to treatment failure (TTF) was defined as the time interval from initial pathological diagnosis of HGT1 till the time to progression to MIBC, metastasis or death.

**Table 1: NMIBC Cohort characteristics (HGT1 n=88)**

| | N | % |
|---|---|---|
| Gender | | |
| Male | 75 | 85 |
| Female | 12 | 14 |
| Unknown | 1 | 1 |

| Microstaging | | |
|---|---|---|
| T1a | 26 | 30 |
| T1b | 60 | 68 |
| Unknown | 2 | 2 |

| Pattern | | |
|---|---|---|
| Papillary | 55 | 63 |
| Solid±papillary | 31 | 35 |
| Unknown | 2 | 2 |

| Multifocality | | |
|---|---|---|
| Single | 39 | 44 |
| Multiple | 45 | 51 |
| Unknown | 4 | 5 |

| Vascular permeation | | |
|---|---|---|
| No | 73 | 83 |
| Yes | 11 | 13 |
| Unknown | 4 | 5 |

| Cis | | |
|---|---|---|
| No | 56 | 64 |
| Yes | 28 | 32 |
| Unknown | 4 | 5 |

| Prostate Cis | | |
|---|---|---|
| No | 76 | 86 |
| Yes | 9 | 10 |
| Unknown | 3 | 3 |

| Size | | |
|---|---|---|
| <3cm | 41 | 47 |
| >3cm | 43 | 49 |
| Unknown | 4 | 5 |

| Squamous metaplasia | | |
|---|---|---|
| No | 57 | 65 |
| Yes | 28 | 32 |
| Unknown | 3 | 3 |

| Micropapillary | | |
|---|---|---|
| No | 64 | 74 |
| Yes | 23 | 26 |
| Unknown | 1 | 1 |

### 1.2. Pathology Review and Annotation

4 µm sections were prepared from archival formalin-fixed paraffin-embedded (FFPE) tissue blocks and stained for H&E. Pathology review of tumor regions of interest (ROI), where tumor cellularity was in excess of 70% were annotated. Up to 5 x 0.6 mm cores were punched from the FFPE tissue blocks within the tumor-rich ROI, as identified from H&E annotated by pathologist (JBa). (See 1.3. below)

### 1.3. RNA extraction, QC and quantification from archival FFPE tissue specimens

The AllPrep FFPE Kit (Qiagen, Germantown, Maryland, USA) was utilized for the RNA extraction. RNA isolates were eluted in a 14 µl volume of RNase/DNase-free H₂O. RNA was quantified utilizing the Quant-iT RiboGreen assay (Life Technologies - cat# R11490). RNA degradation and fragment size was assessed on a Bioanalyzer 2100 instrument (Agilent 5067-1511), using the RNA 6000 nano kit (Agilent 5067-1511). The bioanalyzer DV200 measurement, which calculates the percentage of RNA fragments larger than 200nt, was determined. The percentage of RNA fragment as determined was utilized to infer the RNA input volume for the subsequent library preparation.

### 1.4 Automated RNA-Seq library preparation and sequencing:

50 ng input total RNA was utilized for RNA-Seq library preparation utilizing the TruSeq Stranded RNA Access Library Prep Kit (cat# RS-301-2001). The method was automated on the Biomek® FXP Laboratory Automation Workstation (Beckman Coulter). cDNA libraries were quantified utilizing the Quanti-iT PicoGreen assay (Life Technologies - cat# P7589). Libraries were also quality-checked by Agilent Bioanalyzer using the High Sensitivity DNA kit (cat# 5067-4626). cDNA libraries were sequenced on the Illumina NextSeq500 platform as 75bp paired end reads. The STAR RNA sequencing alignment tool (Spliced Transcripts Alignment to a Reference) aligner [STAR_2.5.0a]) was utilized to align the data to the genome, Homo sapiens UCSC hg19 (RefSeq gene annotations). DeSeq2 was used to perform differential expression analysis (±2-fold, adjP value<0.05). Overlaps were computered between inventor's DE gene set and the annotated Hallmark gene sets in the Molecular Signature DataBase, MSigDB (top 100, FDR q-value<0.05). Gene Cluster 3.0 was used to perform hierarchical clustering. Java TreeView (Version 1.1.5) software was used to plot the heatmap from hierarchical clustering results.

### 1.5. Correlation with the 5 RNA signature subtypes described in TCGA 2017

The present inventors observed 2 major clusters, A (64%, 56/88) and B (36%, 32/88) with some differentiation within B, B1 (25%, 22/88) and B2 (11%, 10/88). The majority of tumors in Cluster A showed low or no expression of immune and neuronal genes. There was also a lack of EMT genes expressed in this cluster, which represent the luminal-infiltrated molecular subtype, so Cluster A could be considered a less aggressive phenotype based on Robertson gene classification. This could be equivalent to GS1 in the Uromol paper (Hedegaard J. et al., 2016). Approximately 70% of tumors in Cluster A had moderate/high expression of basal-squamous markers, with the remaining 30% of tumors showing moderate/high expression of luminal and luminal-papillary markers. Cluster B was enriched for tumors where high expression of the 3 luminal molecular subtypes, luminal (53%), luminal-papillary (66%) and luminal-infiltrated (66%), coupled with the low/absence of expression of the basal-squamous subtype was observed. In contrast to Cluster A, immune and neuronal genes had moderate/high expression. Cluster B2 was differentiated from Cluster B1 based on lower expression of the luminal molecular subytpe genes, such as PPARG, FOXA1, SNX31 and FGFR2 in the latter. Cluster B could be considered a more aggressive phenotype than Cluster A based on Robertson gene classification (Robertson A. G., *et al.* 2017) with the most prominent aggressive markers (infiltrated-luminal) more highly expressed in Cluster B2. This B2 cluster correlates with the class 2 in Uromol (12) Cluster B1 was enriched for HGT1 NMIBC tumors harboring the MP variant (44%, 10/23), followed by Cluster B2 (34%, 8/23) and Cluster A (22%, 5/23).

### 1.6. Statistical Analyses:

Patient and clinical characteristics were summarized as numbers and percentages. Associations between clinical variables (clinicopathologic features and TTF) and gene expression levels were summarized using Wilcoxon's rank sum test. Log rank test was used to summarize the association between gene expression (dichotomized at median) and time to progression (TTP) in univariate analysis. Cox regression model was used to assess the association between gene expression levels adjusting for microstaging (pT1a vs pT1b) (Warrick J. I. et al., 2019) and micropapilary pattern. Hazard ratios and 95% CIs were reported as well.

### 2. Results

Whole transcriptome sequencing (WTS) was performed on RNA extracted from a cohort of 88 HGT1 NMIBC of which, 23 were pathologically annotated as micropapillary HGT1 (MP-HGT1) (Table 1 above).

More than 3000 genes were found to be differentially expressed in MP-HGT1 as compared with conventional urothelial HGT1. The gene expression pattern for MP-HGT1 was more homogeneous than that of HGT1, which is known to exhibit heterogenous biology. Upon application of cut-offs (±2-fold change, AdjP-value<0.05), 523 genes were found to be differentially expressed. A volcano plot showed the most up- and downregulated genes, factoring in both biological and statistical significance.

Enrichment analysis was performed using the MSigDB Hallmark gene set and plotted the statistically significant (FDR<0.05) gene sets associated with the genes that were found to be upregulated in MP-HGT1 (Table 1). We found that MP-HGT1 tumors were enriched with immune system, cell cycle and metabolic pathways, of which the most enriched hallmark gene set was the complement pathway.

Using biological and statistical cut-offs of 2-fold differential expression (between the median of the expression found in the population having micropapillary variant and the median expression found in the population not having micropapillary variant) and adjP value less than 0.05, a 26-gene signature describing MP-HGT1 was found (Table 2) that is enriched for genes associated with metabolism and metabolic transport, immune response, extracellular matrix and osteoblast differentiation.

**Table 2: 26-gene signature that defines MP HGT1 MPBC in NMIBC cohort (Yes= micropapillary, No= non-micropapillary)**

| **Gene** | **Description** | **# Patients** | | **Median (Normalized (Expression)** | | **Log₂FC** | **P-value** |
|---|---|---|---|---|---|---|---|
| | | **Yes** | **No** | **Yes** | **No** | | |
| COL4A4 | collagen, type IV, alpha 4 | 57 | 21 | 0.118 | 2.027 | 4.099 | <0.0001 |
| LCN2 | lipocalin 2 | 58 | 23 | 4.057 | 43.073 | 3.408 | <0.0001 |
| KL | Klotho | 57 | 19 | 0.193 | 1.992 | 3.369 | <0.0001 |
| ABCA13 | ATP-binding cassette, sub-family A (ABC1), member 13 | 62 | 23 | 0.769 | 6.688 | 3.121 | <0.0001 |
| FABP3 | fatty acid binding protein 3 | 61 | 21 | 2.262 | 14.760 | 2.706 | <0.0001 |
| CYP1B1 | cytochrome P450 family 1 subfamily B member 1 | 64 | 23 | 0.028 | 0.125 | 2.168 | <0.0001 |
| ANPEP | alanyl aminopeptidase | 63 | 23 | 1.067 | 2.960 | 1.472 | <0.0001 |
| DACH2 | dachshund family transcription factor 2 | 64 | 23 | 0.053 | 0.004 | -3.585 | <0.0001 |
| KRTAP5-9 | keratin-associated protein 5-9 | 64 | 23 | 0.355 | 0.022 | -4.018 | <0.0001 |
| CAPNS2 | calpain small subunit 2 | 64 | 23 | 0.618 | 0.021 | -4.887 | <0.0001 |
| KRTAP5-8 | keratin-associated protein 5-8 | 64 | 23 | 0.420 | 0.009 | -5.576 | <0.0001 |
| NPAP1 | nuclear pore associated protein 1 | 64 | 23 | 0_{.}040 | 0.001 | -5.637 | <0.0001 |
| GDPD2 | glycerophosphodiester phosphodiesterase domain containing 2 | 64 | 23 | 0.212 | 0.004 | -5.689 | <0.0001 |
| HECTD2-AS1 | HECTD2 antisense RNA 1 | 64 | 23 | 0.091 | 0.000 | -9.827 | <0.0001 |
| SNHG8 | small nucleolar RNA host gene 8 | 64 | 23 | 0.114 | 0.000 | -10.148 | <0.0001 |
| GABBR2 | gamma-aminobutyric acid (GABA) B receptor, 2 | 57 | 23 | 0.484 | 6.182 | 3.675 | 0.0001 |
| C12orf75 | chromosome 12 open reading frame 75 | 61 | 21 | 3.689 | 20.363 | 2.465 | 0.0003 |
| PKP1 | plakophilin 1 | 64 | 23 | 0.364 | 0.055 | -2.727 | 0.0006 |
| CEACAM6 | carcinoembryonic antigen-related cell adhesion molecule 6 | 57 | 23 | 2.124 | 9.993 | 2.234 | 0.0009 |
| CRTAC1 | cartilage acidic protein 1 | 63 | 18 | 12.147 | 0.983 | -3.627 | 0.0014 |
| CD36 | platelet glycoprotein IV | 64 | 23 | 0.128 | 0.683 | 2.421 | 0.0032 |
| IGF2 | insulin-like growth factor 2 | 64 | 23 | 3.876 | 0.483 | -3.004 | 0.0038 |
| MMP7 | matrix metallopeptidase 7 | 51 | 21 | 1.847 | 15.275 | 3.048 | 0.0085 |
| RAET1E | retinoic acid early transcript 1E | 60 | 10 | 4.012 | 1.036 | -1.954 | 0.0124 |
| CHST3 | carbohydrate sulfotransferase 3 | 64 | 23 | 0.820 | 1.530 | 0.900 | 0.0394 |
| TNFRSF11B | TNF receptor superfamily member 11b | 64 | 23 | 0.032 | 0.084 | 1.378 | 0.0662 |

To assess the potential clinical relevance of this 26-gene signature, it was evaluated whether the level of expression was associated with time to progression (TTP) by Log rank. Expression levels were dichotomized into low and high using the overall patient median as the cut-off for each one of the genes. Table 3 listed individual genes showing a statistically significant association with TTP. It was found that high CD36 expression (p=0.002) and low expression of RAETE1 (p=0.0005, Fig 2C) were associated with shorter TTP in univariate analysis. There was trend of higher expression of FABP3 (p=0.13) associated with shorter TTP.

**Table 3: Univariate and multivariate analyses of FABP3, CD36 and RAET1E association with TTP in NMIBC micropapillary variant cohort, where expression was defined as high (>Median) or low (≤Median)**

| | **Expression** | **Progression** | **Univariate Analysis** | | | **Multivariate Analysis** | | |
|---|---|---|---|---|---|---|---|---|
| | | | **HR** | **95% Cl** | **p-value** | **HR** | **95% Cl** | **p-value** |
| FABP3 | High | 12/36 | 1 (ref) | | | 1 (ref) | | |
| | Low | 9/41 | 0.56 | 0.21-1.49 | 0.13 | 0.33 | 0.11-0.97 | 0.045 |
| CD36 | High | 18/40 | 1 (ref) | | | 1 (ref) | | |
| | Low | 6/42 | 0.20 | 0.06-0.61 | 0.002 | 0.31 | 0.08-1.15 | 0.08 |
| RAET1 E | High | 2/34 | 1 (ref) | | | 1 (ref) | | |
| | Low | 14/34 | 0.86 | 1.88-36.40 | 0.0005 | 14.14 | 1.74-114.85 | 0.01 |

In the column of "Progression" the denominator expresses the number of samples analysed which show a high or low expression of the marker; whereas the numerator expresses the number of samples from those cases wherein there have been a progression of the disease.

Multivariate analysis confirmed that high expression of FABP3 was independently associated with shorter TTP (HR: 0.33; 95% CI, 0.11-0.97; P=0.045); as was low expression of RAET1E (HR: 14.14; 95% CI; 1.74-114.85; P=0.01). High expression of CD36 was also associated with a trend towards shorter TTP, though this did not reach statistical significance (HR: 0.31; 95% CI, 0.08-1.15; P=0.08).

A combination of all 3 genes was significantly associated with a shorter TTP (P<0.0001). The analysis of these 3 differentially expressed genes (FABP3, CD36 and RAET1E) allowed to create a risk index that could help to identify patients with MP HGT1 with a higher risk of progression (i.e., with a shorter TTP), in which RC could be recommended as a first treatment option.

### Citation List

### Non Patent Literature

Burtis C. A. et al., 2008, Chapter 14, section "Statistical Treatment of Reference Values;
Hedegaard J. et al., "Comprehensive Transcriptional Analysis of Early-Stage Urothelial Carcinoma", Cancer Cell, 2016; 30(1):27-42;
Kamat A. M. et al., "Micropapillary bladder cancer: a review of the University of Texas M. D. Anderson Cancer Center experience with 100 consecutive patients", Cancer, 2007, 110(1):62-7;
Robertson AG, et al. "Comprehensive Molecular Characterization of Muscle-Invasive Bladder Cancer" Cell. 2017;171(3):540-56.e25;
Spaliviero M et al., "Clinical outcome of patients with T1 micropapillary urothelial carcinoma of the bladder", J. Urol., 2014, 192(3):702-7;
Sui W. et al., "Micropapillary Bladder Cancer: Insights from the National Cancer Database", Bladder Cancer, 2016, 2(4):415-23; and
Warrick J. I. et al., "Intratumoral Heterogeneity of Bladder Cancer by Molecular Subtypes and Histologic Variants", Eur. Urol., 2019; 75(1):18-22.

## Claims

1. An *in vitro* method for the diagnosis and/or the prediction of the progression of high grade T1 (HGT1) micropapillary bladder cancer (MPBC) in a subject, the method comprising quantifying the expression product of each one of FABP3, CD36 and RAET1E genes in an isolated sample from the subject.

2. The *in vitro* method according to claim 1, wherein it is further quantified at least an expression gene product selected from the following list: COL4A4, LCN2, KL, ABCA13, CYP1B1, ANPEP, DACH2, KRTAP5-9, CAPNS2, KRTAP5-8, NPAP1, GDPD2, HECTD2-AS1, SNHG8, GABBR2, C12orf75, PKP1, CEACAM6, CRTAC1, IGF2, MMP7, CHST3, TNFRSF11B, and combinations thereof.

3. The *in vitro* method according to claim 2, wherein it is further quantified the expression gene product from COL4A4, LCN2, KL, ABCA13, CYP1B1, ANPEP, DACH2, KRTAP5-9, CAPNS2, KRTAP5-8, NPAP1, GDPD2, HECTD2-AS1, SNHG8, GABBR2, C12orf75, PKP1, CEACAM6, CRTAC1, IGF2, MMP7, CHST3 and TNFRSF11B genes.

4. The *in vitro* method according to any one of claims 1 to 3, which further comprises the steps of comparing the expression of each one of the gene product(s) with a reference value.

5. The *in vitro* method according to claim 4, wherein the expression gene product is compared with a reference value, and the subject is diagnosed as having HGT1 MPBC if the expression gene product for each one of the markers is at least 2-fold higher with respect to the corresponding reference value.

6. The *in vitro* method according to claim 4, wherein the gene expression product of each one of FABP3, CD36 and RAET1E is compared with the corresponding reference value, and the subject is identified as having bad prognosis when the gene expression products of FABP3 and CD36 are higher and the gene expression product of RAET1E is lower, when compared to the respective reference values.

7. An *in vitro* method for deciding or recommending a radical cystectomy-based medical regimen to a patient with HGT1 MPBC, the method comprising the steps of (i) quantifying the gene expression product of FABP3, CD36 and RAET1E in an isolated biological sample of the patient, (ii) comparing the amount of each expression product with a reference value for each one of the markers, and (iii) recommending a radical cystectomy if the gene expression products of FABP3 and CD36 are higher than the corresponding reference values, and the gene expression product of RAET1E is lower than the corresponding reference value.

8. An *in vitro* method for deciding or recommending a medical regimen to a patient with HGT1 MPBC, the method comprising the steps of (i) quantifying the gene expression product of FABP3, CD36 and RAET1E in an isolated biological sample of the patient, (ii) comparing the amount of each expression product with the reference value for each one of the markers, and (iii) recommending a therapy selected for standard transurethral resection (TURBT)and intravesical bacillus Calmette-Guerin (BCG) therapy, if the gene expression product of FABP3 or CD36 is equal or lower than the corresponding reference value, and/or the gene expression product of RAET1E is equal or higherthan the corresponding reference value.

9. The *in vitro* method according to any one of claims 7 or 8, wherein it is quantified at least one expression gene product from a gene selected from the following list: COL4A4, LCN2, KL, ABCA13, CYP1B1, ANPEP, DACH2, KRTAP5-9, CAPNS2, KRTAP5-8, NPAP1, GDPD2, HECTD2-AS1, SNHG8, GABBR2, C12orf75, PKP1, CEACAM6, CRTAC1, IGF2, MMP7, CHST3, TNFRSF11B, and combinations thereof.

10. The *in vitro* methods according to any one of claims 1 to 9 wherein the gene expression product is mRNA.

11. The *in vitro* method according to any one of claims 1 to 10 wherein the gene expression product is quantified by a fluorescence-based technique.

12. The *in vitro* method according to any one of claims 1 to 11, wherein the sample is an isolated bladder tissue sample.

13. A combined use of FABP3, CD36 and RAET1E as a marker of diagnosis, prediction of progression of HGT1 MPBC or of deciding or recommending a medical regimen to a patient with HGT1 MPBC.

14. Use of means for quantifying the gene expression product of each one of FABP3, CD36 and RAET1E in any of the methods as defined in any of the claims 1-12.

15. The use of means according to any one of claim 14 wherein said means is a fluorescent RNA-binding dye.
